# EUROPEAN PATENT APPLICATION

(11) **EP 4 670 546 A1**
(43) Date of publication of application: **31.12.2025**
(21) Application number: 24760446.5
(22) Date of filing: 22.02.2024
(51) Int. Cl.: A42B 1/017, A42B 1/00, A42B 1/019, A42B 1/041

(54) **CAP**

(30) Priority: 22.02.2023 JP 2023026642
(71) Applicant: RELIVE Co., Ltd., Sendai-shi Miyagi 981-3212 (JP)
(72) Inventor: SASAKI, Takashi, Sendai-shi Miyagi 981-3212 (JP)
(74) Representative: HKW Intellectual Property PartG mbB
(86) International application number: PCT/JP2024/006617
(87) International publication number: WO 2024/177148

(57) **Abstract**

Issue: Making available a cap that can improve the wearer's physical functioning as well as flexibility.

Resolution Means: A cap 100 of the present invention comprises a cap body element 10 for covering an area of the head, and in the cap body element 10 a head-area patterned piece 20 extending along a centerline (C) of the head area. The head-area patterned piece 20 is constituted from a tape medium 21 adhered to at least one of either the reverse surface or the obverse surface of the cap body element 10. A mineral(s) (30) is then contained in the tape medium 21 in the head-area patterned piece 20.

## Description

### TECHNICAL FIELD

The present invention relates to caps. It especially relates to caps that can improve the wearer's physical functioning and flexibility besides.

For the record, the present application claims right of priority based on JP 2023-026642 A, filed February 22, 2023; the entire contents of that application are incorporated into the present specification by way of reference.

### BACKGROUND ART

Athletic wear for improving athletic performance when sports and the like are engaged in has become familiar in recent years. Needs with respect to athletic wear include some that are extraordinarily deep-rooted. To take the example of golf: users who are thinking they would like to raise their club-head speed and extend their flight distance even without daily strength training and stretching are conspicuously prevalent (see Patent Document 1, for example).

### PRECEDENT TECHNICAL LITERATURE

### PATENT DOCUMENTS

Patent Document 1: JP 4061336 B
Patent Document 2: JP 2004 - 210758 A

### SUMMARY OF INVENTION

### ISSUES INVENTION IS TO RESOLVE

The present applicant, hitting upon the idea that rather than conventional athletic wear, simply putting headgear (a cap) on the head could improve physical functioning/flexibility, arrived at the present invention.

The "Ionic Far-Infrared Cap" disclosed in Patent Document 2 is an instance of conventional headgear (a cap). This cap has a configuration in which, with their source material being weakly radioactive minerals as well as far-infrared radioactive minerals, ceramic tiles into which the material has been worked are furnished inside the cap. As determined by this ionic far-infrared cap, the negative ions and far infrared can yield benefits of protecting the scalp and fostering scalp hair growth, less any concern of side effects arising along with the medicinal-like benefits.

While the ionic far-infrared cap disclosed in Patent Document 2 can yield benefits of protecting the scalp and fostering scalp hair growth, the cap lacks function that would improve physical functioning/flexibility.

A principal object of the present invention, which has been brought about taking such points into consideration, is to make available headgear (a cap) that can improve the wearer's physical functioning and flexibility besides.

### MEANS FOR RESOLVING THE ISSUES

A cap involving the present invention is a cap that is worn on the crown, and comprises a cap body element for covering an area of the head, and in the cap body element a head-area patterned piece extending along a centerline of the head area. The head-area patterned piece is constituted from a tape medium adhered to at least one of either the reverse surface or the obverse surface of the cap body element. The tape medium in the head-area patterned piece contains a mineral(s).

With a preferred embodying mode, the mineral(s) is at least one type of mineral selected from the group consisting of radium ore, germanium, quartz, "terahertz" ore, and tourmaline. The mineral(s) is invested into the tape medium by being printed onto it. The mineral(s) invested by the printing have a pattern form selected from the group consisting of geometric shapes, design marks, and written characters.

With a preferred embodying mode, on one region of the tape medium a first patch(es) constituted from a first metal is formed. On another region of the tape medium a second patch(es) constituted from a second metal is formed. The first patch(es) is a gold patch(es), and therein the second patch(es) is a silver patch(es).

With a preferred embodying mode, on one region of the tape medium a first patch(es) constituted from a first metal is formed. On another region of the tape medium a second patch(es) constituted from a second metal is formed. The first patch(es) is either a patch(es) composed of an alloy of zinc and copper, or a copper patch(es), and therein the second patch(es) is an aluminum patch(es).

In a preferred embodying mode, the head-area patterned piece is formed on the reverse face of the cap body element.

In a preferred embodying mode, the head-area patterned piece has a rectangular form. A rim part that defines an opening on the head-area patterned piece constitutes an elastic section. On the cap body element, a mesh section that extends along the head-area centerline is formed. The head-area patterned piece is formed on an inner-surface side of the mesh section.

In a preferred embodying mode, the cap body element is constituted from a stretchable material. The width of the head-area patterned piece is 25 mm ±10 mm. The head-area patterned piece is formed in such a way as to be symmetrical with respect to the head-area centerline.

Another cap involving the present invention is a cap that is worn on the crown, and comprises a cap body element for covering an area of the head, and in the cap body element a head-area patterned piece extending along a centerline of the head area. The head-area patterned piece is constituted from an ink-impressed figure(s) formed on at least one of either the reverse surface or the obverse surface of the cap body element (10). The ink-impressed figure(s) contains powder of a mineral(s).

With a preferred embodying mode, the mineral(s) is at least one type of mineral selected from the group consisting of radium ore, germanium, quartz, "terahertz" ore, and tourmaline. The ink-impressed figure(s) has a pattern form selected from the group consisting of geometric shapes, design marks, and written characters.

With a preferred embodying mode, on one region of the head-area patterned piece a first patch(es) constituted from a first metal is formed. On another region of the head-area patterned piece a second patch(es) constituted from a second metal is formed. The first patch(es) is a gold patch(es), and therein the second patch(es) is a silver patch(es).

With a preferred embodying mode, on one region of the head-area patterned piece a first patch(es) constituted from a first metal is formed. On another region of the head-area patterned piece a second patch(es) constituted from a second metal is formed. The first patch(es) is either a patch(es) composed of an alloy of zinc and copper, or a copper patch(es), and therein the second patch(es) is an aluminum patch(es).

In a preferred embodying mode, the head-area patterned piece is formed on the reverse face of the cap body element. The cap body element is constituted from a stretchable material. The width of the head-area patterned piece is 25 mm ±10 mm. The head-area patterned piece is formed in such a way as to be symmetrical with respect to the head-area centerline.

A separate cap involving the present invention is a cap that is worn on the crown, and comprises a cap body element for covering an area of the head, and in the cap body element a section of head-area patternings arrayed along a centerline of the head area. The head-area patternings section has mineral buttons provided on at least one of either the reverse surface or the obverse surface of the cap body element. The mineral buttons are plurally arrayed along the head-area centerline.

In a preferred embodying mode, the mineral buttons are formed on the reverse face of the cap body element. The mineral buttons are circular articles in the interiors of which a mineral(s) is housed. The mineral buttons are arrayed evenly spaced apart along the centerline of the head area.

A method, involving the present invention, of manufacturing a cap comprises a step of preparing a cap a body element for covering an area of the head, and a step of forming, in the head-area-covering cap body element along a centerline of the head area, head-area patterned pieces in which a mineral(s) is contained.

With a preferred embodying mode, along the centerline the adhering of a tape medium in which the mineral(s) is contained is carried out.

With a preferred embodying mode, in the step of forming the head-area patterned pieces, the forming of an ink-impressed figure(s) by applying an ink in which the mineral(s) is contained is carried out.

With a preferred embodying mode, in the step of forming the head-area patterned pieces, the plural-item forming along the centerline of mineral buttons in the interiors of which the mineral(s) is housed is carried out.

With a preferred embodying mode, further, on one region of the head-area patterned pieces the forming of a first patch(es) constituted from a first metal, and then on another region of the head-area patterned pieces the forming of a second patch(es) constituted from a second metal are carried out.

### EFFECTS OF INVENTION

As defined by a cap of the present invention, head-area patterned pieces extending along a centerline of the cap body element are constituted from a tape medium, wherein the presence of the head-area patternings can promote circulation of cerebrospinal fluid, as a result of which the physical functioning, and flexibility besides, of a wearer (user) donning the cap can be improved.

### BREIF DESCRIPTION OF DRAWINGS

Fig. 1: A perspective view schematically representing the configuration of a cap 100 involving modes of embodying the present invention.
Fig. 2: A front elevation schematically representing the configuration of the cap 100.
Fig. 3: An upper-surface view schematically representing the configuration of the cap 100.
Fig. 4: A diagram schematically representing a head-area patterned piece 20 (tape article 21) of present embodying modes.
Fig. 5: A diagram for explaining muscles of the head.
Fig. 6: A diagram for explaining a head 90 skeletal structure (skull).
Fig. 7 (a) and (b): Views schematically representing, respectively, states in which the skull is dilated (extended) and constricted (contracted).
Fig. 8: A diagram schematically representing a head-area patterned piece 20 (tape article 21) of present embodying modes.
Fig. 9: A diagram for explaining the relationship (flow of magnetism) between a first patch 51 and a second patch 52.
Fig. 10: A table setting forth results of a physical functioning/flexibility test when the cap 100 is being worn.
Fig. 11 (a) and (b): Drawing-substitute photographs showing, respectively, states of forward bends when with and without the cap 100.
Fig. 12 (a) and (b): Drawing-substitute photographs showing, respectively, states of a crouched-stance leap when with and without the cap 100.
Fig. 13 (a) and (b): Drawing-substitute photographs showing, respectively, states of rearward arm swing when with and without the cap 100.
Fig. 14 (a) and (b): Drawing-substitute photographs showing, respectively, states of a trunk lift when with and without the cap 100.
Fig. 15: A table setting forth results of activity-level/stability-level/comfort-level/alertness-level tests when the cap 100 is being worn.
Fig. 16: A diagram illustrating the method of reckoning activity level/stability level/comfort level/alertness level.
Fig. 17: A perspective view schematically representing the configuration of a modified example of a cap 100 involving modes of embodying the present invention.
Fig. 18: A drawing-substitute photograph showing the disposition of mineral buttons 23 in the modified example of a cap 100.

### MODES FOR EMBODYING THE INVENTION

Hereinbelow, referring to the drawings, an explanation of preferred modes of embodying the present invention will be made. Regarding the drawings in the following, for the sake of making the explanation concise, elements and regions exhibiting the same actions are labeled with the same reference marks, and reduplicating description in some instances is either omitted or abbreviated. Furthermore, in every diagram the dimensional relationships (length, width, thickness, etc.) in some cases do not necessarily reflect the actual dimensional relationships accurately. Nevertheless, in designated drawings some dimensional relationships have been made coincident, and in those cases, from the dimensional/positional relationships in each diagram it is possible to derive the six primary views. It will be appreciated that being headgear (a cap), the form and the structure in the six primary views often may be apprehended ignoring thicknesses as being of designated thickness.

Further, particulars that are necessary for implementing the present invention and are matters apart from the particulars specifically referred to in the present specification can be apprehended to be particulars of design for the person skilled in the art, based on conventional technology in the given field. The present invention can be implemented based on the content disclosed by the present specification and drawings, and on common technical knowledge in the given field. What is more, the present invention is not limited to the following embodying modes.

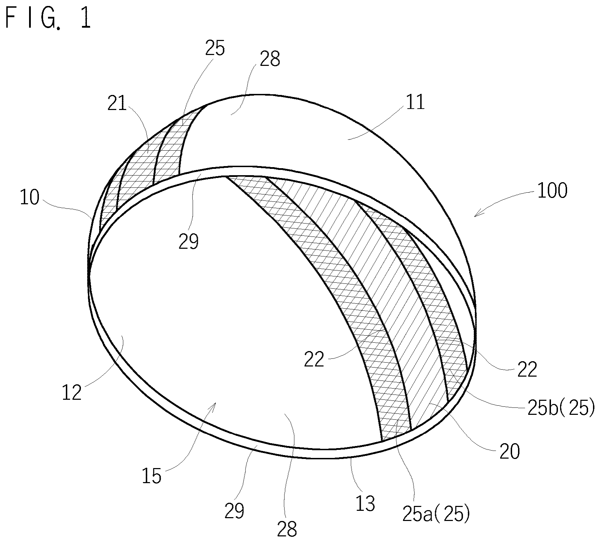

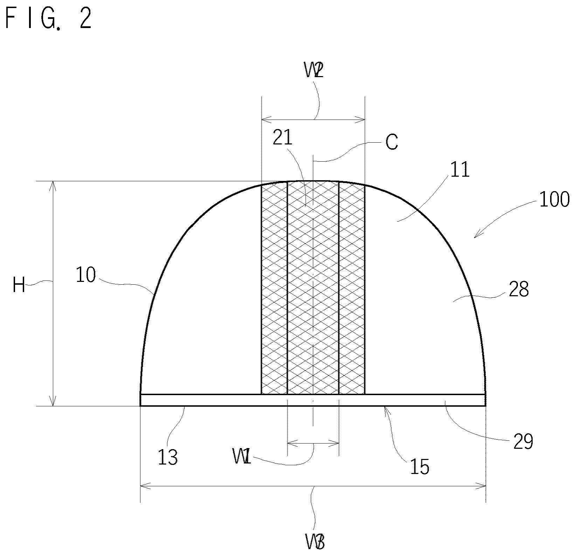

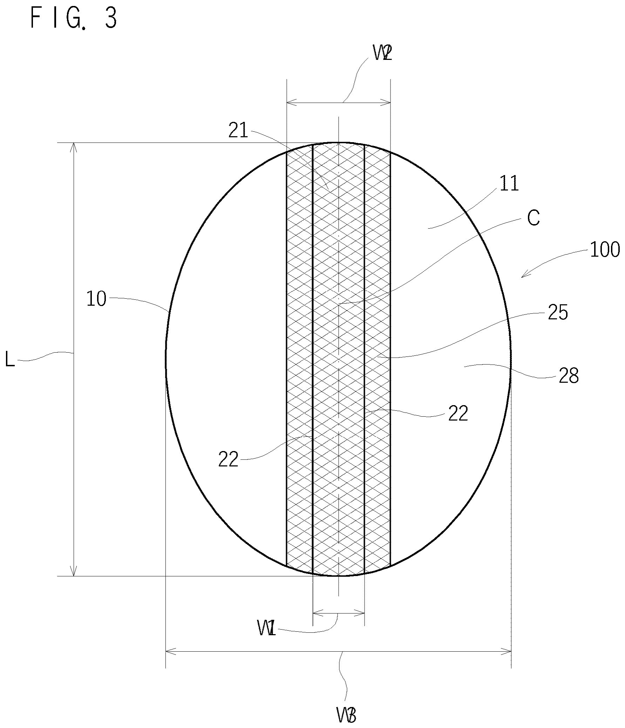

Fig. 1 is a perspective view in which a cap (headgear) 100 involving, respectively, modes of embodying the present invention is seen from the underside. Likewise, Fig. 2 is a diagram (front elevation) in which the cap 100 of the present embodying modes is viewed from the front-elevational side, while Fig. 3 is a diagram (upper-surface view) in which the cap 100 of the present embodying modes is viewed from the upper side.

The cap (headgear) 100 of the present embodying modes, for being worn on the crown, is furnished with a cap body element 10 for covering an area of the head. On the cap body element 10, a head-area patterned piece 20 is formed. As indicated in Fig. 2 and Fig. 3, the head-area patterned piece 20 extends along a centerline (C) of the head area. The head-area patterned piece 20 is constituted from a tape medium (21) adhered to at least one of either the reverse surface (12) or the obverse surface (11) of the cap body element 10. Therein, contained in the tape medium (21) along the head-area patterned piece 20 is a mineral(s). The tape medium (21) of the present embodying modes is constituted from cloth (cloth from natural material, otherwise synthetic-fiber cloth), with mineral(s) being either subsumed in the interior of the tape medium (21), or mineral(s) being dispersed in the tape medium (21).

The cap body element 10 of the present embodying modes is something like a swimming cap, such as not to be liable to slip off the head, and has a roughly hemispherical or semi-ovoid shape. The cap body element 10, furthermore, is constituted from a material having stretchability. And with the configurational example diagrammed, the head-area patterned piece 20 is formed on the reverse surface of the cap body element 10. The head-area patterned piece 20 of the present embodying modes is, furthermore, of rectangular form. The outer edges (left side/right side) 22 that define the shape of the head-area patterned piece 20 are rectilinear, and the head-area patterned piece 20 extends from one end to the other end of the reverse surface 12 of the cap body element 10. The head-area patterned piece 20 is formed in such a way as to be symmetrical with respect to the centerline (C) of the head area. With the configuration example of the present embodying modes, the head-area patterned piece 20 is anchored by sewing it on with thread to the reverse surface of the cap body element 10.

With the configuration of the present embodying modes, furthermore, as represented in Fig. 1, a rim part (mouth rim part) 13 that defines the opening 15 on the head-area patterned piece 20 constitutes an elastic section 29. The elastic section 29 enables firmly fixing the cap 100 to the head area of someone using it (the user). The elastic section 29 can be formed by processing the fabric in the region of the rim part 13 of the cap body element 10 into a hollow structure and threading rubber fibers (rubber cords) therethrough. Also, the elastic section 29 can be fabricated by attaching (fixing) to the rim part 13 of the cap body element 10 a material constituted from a rubber stuff (a rubber linear component or a rubber cord component).

With the configuration example of the present embodying modes, on the cap body element 10, a mesh section 25 that extends along the head-area centerline (C) is formed. Therein, the head-area patterned piece 20 is formed on the middle of the mesh section 25. In concrete terms, the head-area patterned piece 20 is formed in the mid-region on the inner-surface (12) side of the mesh section 25. With the example depicted in Fig. 1, the head-area patterned piece 20 is formed on the inner surface (12) of the mesh section 25 of the cap body element 10. With this example, as indicated in Fig. 2 and Fig. 3, the width W2 of the mesh section 25 is broader than the width W1 of the head-area patterned piece 20. Accordingly, as indicated in Fig. 1, on the outer side of the outer edges (boundary lines 22) of the head-area patterned piece 20, the mesh section 25 of the margins (25a and 25b) is located. Located externally thereof are regions 28 where in the cap body element 10 the mesh section 25 is not formed. The mesh section 25 being in the middle area of the cap body element 10 facilitates releasing heat (including sweat) from the user's head area to the air outside. It should be noted that the entire cap body element 10 (i.e., the region 28 areas also) may be made the mesh section 25, and also that it is possible to form the head-area patterned piece 20 on the cap body element 10 without providing the mesh section 25.

The head-area patterned piece 20 of the present embodying modes contains a mineral(s) (powder). In one example, a mineral(s) (a mineral that is referred to as a so-called healing crystal (e.g., radium ore, germanium, quartz, "terahertz ore," tourmaline, etc.), or a blend of minerals of several kinds) of 325-mesh particle size (approximately 44 microns) is invested into the head-area patterned piece 20. And as may suit, additives apart from the mineral(s) (powder) may be included. As the mineral(s) (powder), a mineral(s) of at least one kind (or a mineral-powder blend of two or more kinds) selected from the group consisting of radium ore, germanium, quartz, terahertz ore, and tourmaline can be utilized.

The material constituting the cap body element 10 of the present embodying modes is a fabric that is typically employed for garments (particularly, one that is suited to caps), and that as being a cap has suitable stretchability. It should be understood that stretchability herein means what for a cap (garment) is a suitable property, not what especially demands a rubber- (elastomer)-like restorative capacity. As for the material (fabric) constituting the cap body element 10, synthetic-fiber textiles (e.g., nylon, polyurethane, and the like), natural-fiber textiles (e.g., cotton fiber (cottons), silk fiber (silks), and the like), mixed-yarn synthetic-fiber textiles (e.g., nylon-polyurethane blended textiles and the like), or textiles that are blends of natural-fiber textiles and synthetic-fiber textiles (e.g., cotton-polyester mixed yarns and the like), as well as textiles that are blends of a plurality of types (e.g., natural-fiber textile (cotton or the like)/nylon/polyurethane textile, rayon textile/acrylic textile/polyester textile, acrylic textile/polyester textile/rayon textile/polyurethane textile, etc.) can be cited.

Furthermore, in situations where the tape medium 21 constituting the head-area patterned piece 20 is cloth media (herein, cotton (e.g., 40-count combed cotton)), it is preferable that the tape medium 21 be sewn, affixing (anchoring) it to the cap body element 10 so that the tape medium 21 does not come off/slip out of place. Furthermore, with heat-sensitive adhesive (or iron-on adhesive) having been applied to a surface of the cloth that is brought into contact with (fixed to/adhered to) a surface (herein, inner surface) of the cap body element 10 (clothing), by heating the given tape medium with a heater (in this case, an iron), it can be affixed (adhesively anchored) to the cap body element 10 (clothing body element). What is more, anchoring it with iron-on adhesive and then sewing it in with thread is handier. It should be noted that in order to make it so that even laundered the tape medium 21 will not come off/slip out of place, the tape medium may be sewn on and affixed (it may be anchored) to the cap body element 10.

The width W1 of the head-area patterned piece 20 (tape medium 21) of the present embodying modes is, for example, 30 mm or less (with the diagrammed instance, 25 mm ±5 mm, or otherwise on the order of 10 mm to 30 mm, for example). And the width W2 of the mesh section 25 is, for example, 80 mm or less (in the diagrammed instance, 65 mm ±10 mm, for example). Likewise, the height H of the cap body element 10 is, for example, 20 cm or less (in the diagrammed instance, on the order of 15 cm ±5 cm, for example). The dimension W3 (shorter-span diameter) of the cap body element 10 is 25 cm or less (in the diagrammed instance, on the order of 20 cm ±5 cm, for example). The dimension L (longer-span diameter) of the cap body element 10 is, for example, 30 cm or less (in the diagrammed instance, on the order of 22 cm ±5 cm, for example). The width of the elastic section 29 is, for example, 10 mm or less (in the diagrammed instance, on the order of 5 mm to 8 mm, for example). Not limited to being those, nevertheless, these dimensions (for example, W1 of the head-area patterned piece 20) may, as long as beneficial effects are demonstrated, exceed the upper limit value; likewise with the lower limit value-they may fall below it.

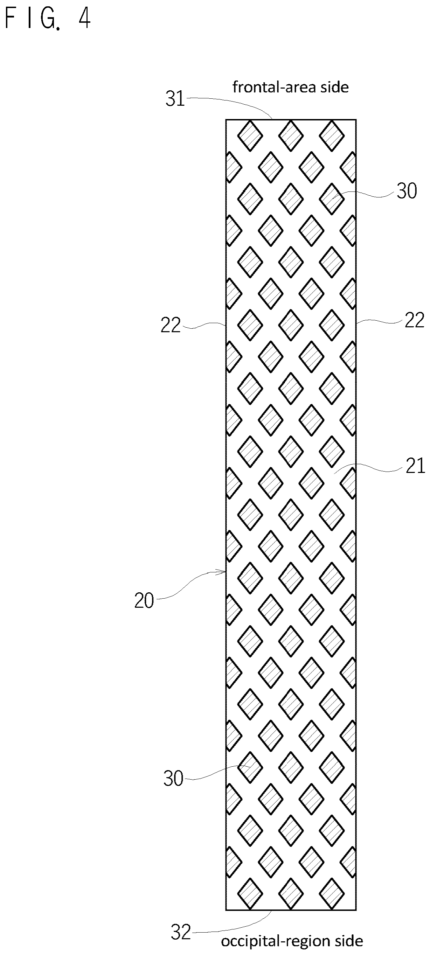

Fig. 4 diagrams one example of the configuration of a head-area patterned piece 20 (tape medium 21) of the present embodying modes. In this example, one end (one side) 31 of the head-area patterned piece 20 (tape medium 21) is situated along the frontal area, while the other end (the other side) 32 of the head-area patterned piece 20 (tape medium 21) is situated along the occipital region. With the configurational example shown in Fig. 4, the mineral(s) contained in the head-area patterned piece 20 are present as design-mark patternings 30 invested into it by being printed on. In concrete terms, a powder of at least one kind (or an ore powder blend of two or more kinds) of mineral selected from the group consisting of, for example, radium ore, germanium, quartz, terahertz ore, and tourmaline is compounded into an ink, and the mineral-containing ink is printed onto the tape medium 21, investing the mineral(s) into it. The mineral patternings 30 invested by printing have a pattern form that is geometric shapes, design marks, written characters, or a complex of these. With the example shown in Fig. 4, the ore patternings 30 are squarish (e.g., rhomboidal), but they can be rendered in patterns apart from ones of these sorts. The ore patternings 30 may be of geometric shapes such as circles (including ellipses and ovals), hexagons, or stars, or may be design marks such as waves, whorls, arabesque designs, checkered designs, floral patterns, animal patterns, landscape patterns, and cartoon/animation character patterns. The ore patternings 30 may be written characters such as alphabetic letters, numeric figures, symbols, written Japanese, Chinese characters, or Hangul. While regularly arrayed ore patternings 30, being more attractive, are preferable, they may be in an irregular array as may suit. It should be noted that in the head-area patterned piece 20 (tape medium 21) of the present embodying modes, the mineral(s) may be introduced in a mode such as to compound (or impregnate) the mineral(s) (powder) into the entirety of the tape medium 21. Also, the mineral(s) may be introduced in a mode such that the mineral(s) (powder) is compounded (or impregnated) into the interior of (hollows in) the head-area patterned piece 20 (tape medium 21) of the present embodying modes.

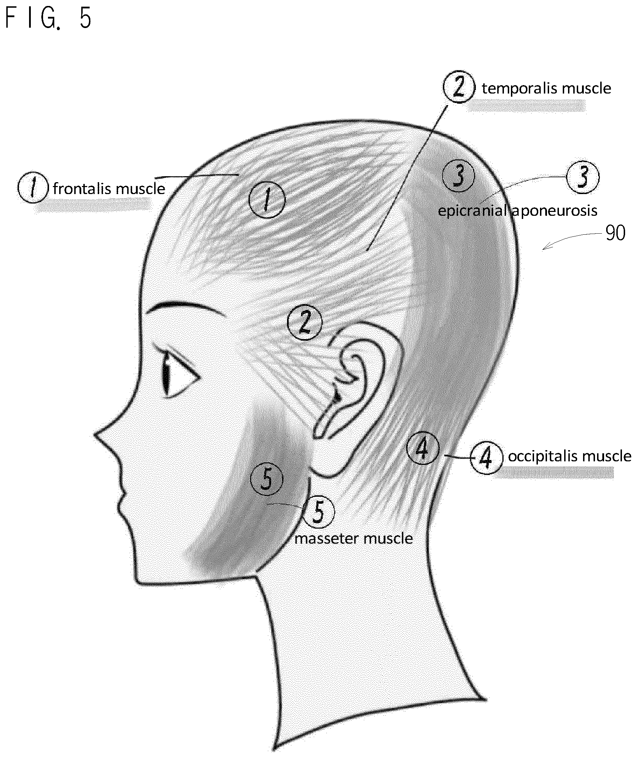

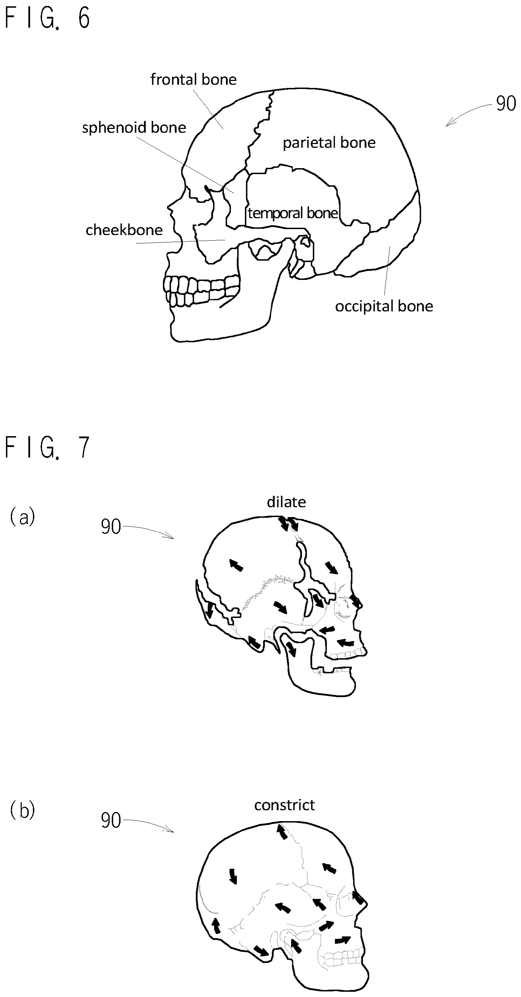

Fig. 5 schematically illustrates muscles in a head 90. The muscles of the head include: (1) the *frontalis* muscle, (2) the *temporalis* muscle, (3) the *epicranial aponeurosis*, (4) the *occipitalis* muscle, and (5) the *masseter* muscle. On the exterior of the muscles of the head 90, the bones of the head 90 are (the skull is) situated. Fig. 6 schematically diagrams the skeletal structure of a head 90, depicting, as the bones (skull) of the head 90, the frontal bone, a temporal bone, a parietal bone, the occipital bone, and a cheekbone. Fig. 7 (a) and (b) schematically represent, respectively, states in which the skull is dilated (extended) and constricted (contracted).

The skull consists of the occipital bone, the sphenoid bone, the temporal bones, the frontal bone, and the parietal bones, with the entirety except for the lower cheekbones and the hyoid bone being immovably joined together. Herein, leaving aside the facial bones, to explain focusing on the braincase: in the cranium any number of sutures are present. To say that the sutures are joined immovably only amounts to a broad-strokes description; in the sutures locally they expand or contract, albeit slightly. With a cap 100 of the present embodying modes, being that functionality on the basis of the expansion or contraction of the sutures arises, the presence of the head-area patterned piece 20 in the cap 100 can promote cerebrospinal fluid recirculation. Cerebrospinal fluid fills, apart from the brain interior, the spinal canal as well; and with the spinal nerve roots extending inside the canal, therein, that a cap 100 of the present embodying modes can improve athletic functioning (physical functioning and flexibility besides) is because it can make neurotransmission in the pyramidal tracts and extrapyramidal system fluid.

Herein, the explanation will be continued further on the premise that the human body can produce smooth movement by means of the muscles as a pseudo-fluid structure. This smooth movement also is in that there is movement of the human skeleton. Skeletal movement is based on structural leaning pressure owing to one's center-of-balance base of support compensating with respect to movement from above. Herein the expansion/constriction (contraction) of the skull indicated in Fig. 7 (a) and (b) works in tandem with movement of the diaphragm (muscle), meaning that it is in synchronization with breathing. By the inhaling and exhaling of breath, the diaphragm moves; then, naturally, by the working of the muscles in tandem the inclination of the skeleton changes, whereby the skull moves. To explain further: when with breathing the diaphragm moves, the ilia move in tandem and in response to the breathing a front turn (the ilia curl inward) or a back turn (the ilia open outward) takes place. Then in response to the pelvic movement, the skull expands or contracts its sutures, carrying out fine adjustments to gain gravitational-center balance. Movement along the opposite route also naturally exists. Consequently, the skull shifts the sutures albeit slightly. With healthy movement, when the pelvis opens (the ilia particularly), the skull contracts, while when the pelvis closes (the ilia particularly), the skull enlarges. With unhealthy movement, when the pelvis and skull take on a rhythm in which in synchronization they expand/contract at the same point, exercise/physiology-wise all manner of malaise arises. The human body through breathing is constantly making subtle movements (especially, in this case movements of the skull and ilia). A cap 100 of the present embodying modes, then, can modulate this movement to what feels right.

A cap 100 of the present embodying modes is furnished with the head-area patterned piece 20 that extends along the head-area centerline (C); the presence of this head-area patterned piece 20 can promote cerebrospinal fluid recirculation and also modulate the skull then the ilia to make the gravitational-center balance into what feels right, and thereby can improve physical functioning, as well as flexibility, of the wearer (user) wearing the cap 100. It should be noted that while "taping," in which tape is stretched directly on the body-which especially athletes often use-works to protect muscles, with the technology of the present embodying modes gentle stimulation-in contrast to strong stimulation from affixing tape directly-is availed of, and as it were, thus may be referred to as "noncontact-model indirect taping." It is supposed, then, that differences between the fabric area of the cap body element 10, and the head-area patterned piece 20 (tape medium 21) in texture and other properties (difference in tactile feel, differences in softness, elasticity, surface smoothness, and other properties of the material) are produced, and that the differences in properties, activating given regions, can assist capacity for movement. It should be noted that though the differences in these properties may be slight, it is not that the head-area patterned piece 20 intensely stimulates given locations to accomplish the function of improvement in capacity for movement; (inasmuch as the body is a cluster of delicate sensors) that it occasions stimulation is enough-even if the difference in properties due to the presence of the head-area patterned piece 20 is slight, beneficial effects of improving exercise capacity can be demonstrated. And like it is a fact that acupuncture and moxibustion performed on the head area can have an effect on the body, a cap 100 of the present embodying modes, simply by being worn on the head, can improve the wearer's physical functioning and flexibility besides. There is a likelihood, furthermore, that by a mineral(s) being compounded into the tape medium 21, influences from the mineral(s) (e.g., negative ions, terahertz waves, static electricity, far infrared rays, hormesis, etc.) amount to stimuli, while thereby there is a likelihood that the beneficial effects from a cap 100 of the present embodying modes are reinforced. It should be noted that with a cap 100 of the present embodying modes, as opposed to making the entirety of the cap body element 10 a head-area patterned piece 20, from the viewpoint of noncontact-model indirect taping (or from a view toward cranial-suture vectors and stimulation zones), it is preferable that regions where the head-area patterned piece 20 is not present (e.g., regions 28) exist. To explain further: Comprehensively synthesizing such viewpoints, the head-area patterned piece 20 in a cap 100 of the present embodying modes is formed in such a way as to extend along the centerline (C) of the cap body element 10.

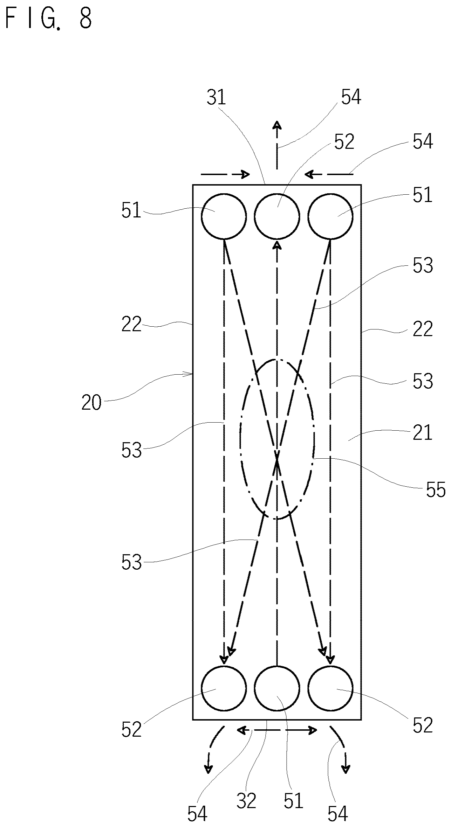

Secondly, with a cap 100 of the present embodying modes, modification as represented in Fig. 8 can be carried out. Fig. 8 schematically represents the configuration of a head-area patterned piece 20 (tape medium 21) in a modified example of a cap 100 of the present embodying modes. It should be noted that in the head-area patterned piece 20 (tape medium 21) depicted in Fig. 8, the above-mentioned minerals (ores) may be compounded, but there can also be implementations in which such minerals (ores) are not compounded.

On a region on one end (herein, the region around the edge part 31 along the frontal area) of the head-area patterned piece 20 (tape medium 21) represented in Fig. 8, first patches 51 constituted from a first metal (in this instance, *kin* (gold)) are formed. Then, in a region on the other end (herein, the region around the edge part 32 along the occipital area) of the head-area patterned piece 20 (tape medium 21), second patches 52 constituted from a second metal (in this instance, *gin* (silver)) are formed. When viewed along the longitudinal direction of the head-area patterned piece 20 (tape medium 21), the first patches (gold patches) 51 and the second patches (silver patches) 52 are arranged in such a way as to form pairs.

With the depicted configuration example, in the region on the one end (the region around the edge part 31 along the frontal area), the first patches (gold patches) 51 and a second patch (silver patch) 52 are arrayed such as to be in alternation. Specifically, they are arrayed in the order, first patch (gold patch) 51, second patch (silver patch) 52, first patch (gold patch) 51. Likewise, in the region on the other end (the region around the edge part 32 along the occipital area), the second patches (silver patches) 52 and a first patch (gold patch) 51 are arrayed such as to be in alternation. Specifically, they are arrayed in the order second patch (silver patch) 52, first patch (gold patch) 51, and second patch (silver patch) 52.

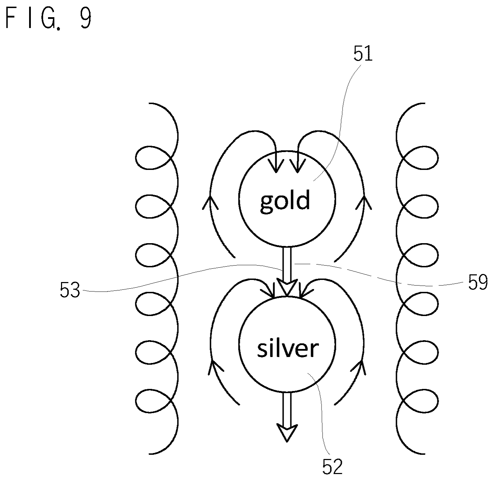

Fig. 9 is a schematic diagram for explaining the relationship between a first patch (gold patch) 51 and a second patch (silver patch) 52. Herein, in that faint electric currents flow through the human body, around the human body magnetic fields are generated. With the configuration of the present embodying mode, as indicated by the arrows 59, free electrons are induced (or else biophotons are generated) through the first patch (gold patch) 51 and led toward the second patch (silver patch) 52, and a magnetic field (arrow 53) from the first patch (gold patch) 51 into the second patch (silver patch) 52 is formed, which can modulate the human body's magnetic fields. Furthermore, in terms of the example represented in Fig. 8, the flow of the magnetic field (arrows 54) from the frontal-area directed edge part 31 toward the frontal area merges with what is known as the Conception Vessel in Oriental medicine. Then, the magnetic-field flow (arrows 54) from the occipital-area directed edge part 32 to the occipital area merges with what is known as the Bladder Meridian in Oriental medicine. The first patches (gold patches) 51 are, speaking from a magnetic viewpoint, tantamount to the S-pole of a magnet; likewise, the second patches (silver patches) 52 are, speaking from a magnetic viewpoint, tantamount to the N-pole of a magnet. In the region of the intervals from the first patches (gold patches) 51 to the second patches (silver patches) 52, a region (55) of substantially zero magnetic field arises as an interference wave between the N-pole and the S-pole. This interference wave (region 55) being brought about is desirable since, with a three-dimensional-axis vector being formed, it leads to promotion of cerebrospinal fluid recirculation taking place.

With the example configuration of the present embodying mode, the first patches (gold patches) 51 and the second patches (silver patches) 52 are rendered circular, but they may be made other shapes (e.g., elliptical, oblong, squarish, hexagonal, etc.). And the number first patches (gold patches) 51 and second patches (silver patches) 52 that are best suited can be adopted. Furthermore, the example configuration of the present embodying mode has the first patches 51 be gold patches and has the second patches 52 be silver patches, but inasmuch as the configuration is not thereby limited, the patches can be made pairings of other, differing kinds of metals. Such a pairing of differing kinds of metals is one that generates magnetic flows (arrows 53 and others) from the first patches 51 to the second patches 52 when a cap 100 of the present embodying modes is worn on a head 90. As an example of this sort of pairing of differing kinds of metals, a pairing of aluminum (tantamount to the N-pole) and a metal consisting of zinc/copper (tantamount to the S-pole) can be given. Likewise, a pairing of yellow copper (an alloy of copper and zinc) and aluminum (100% aluminum metal) can be given. Others that can be cited are a pairing of nickel-yellow copper (an alloy of copper, zinc, and nickel) and aluminum, a pairing of cupronickel (an alloy with copper as the core component and containing from 10% to 30% nickel) and aluminum, and a pairing of copper and aluminum.

### Embodying Examples

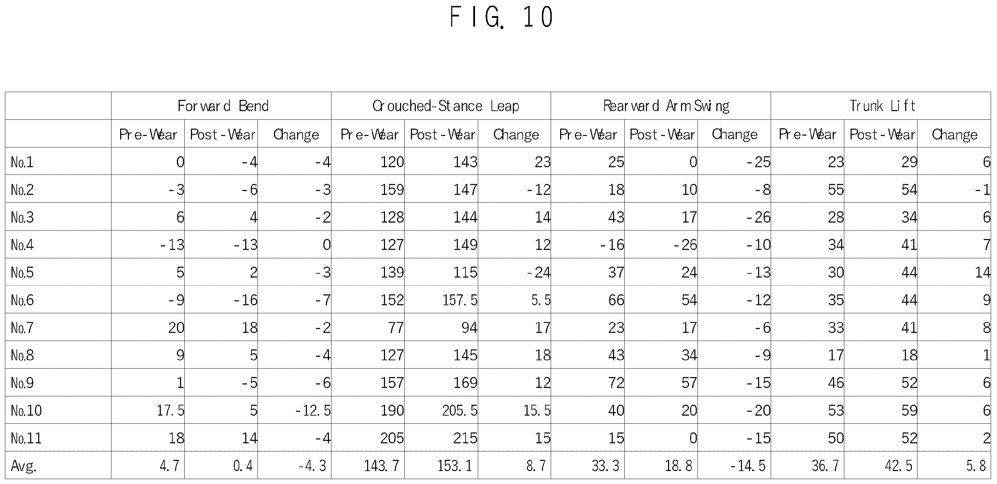

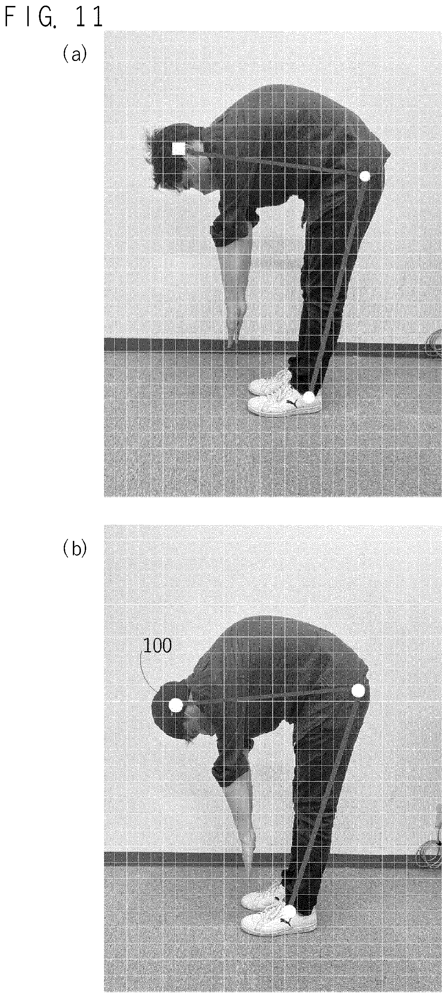

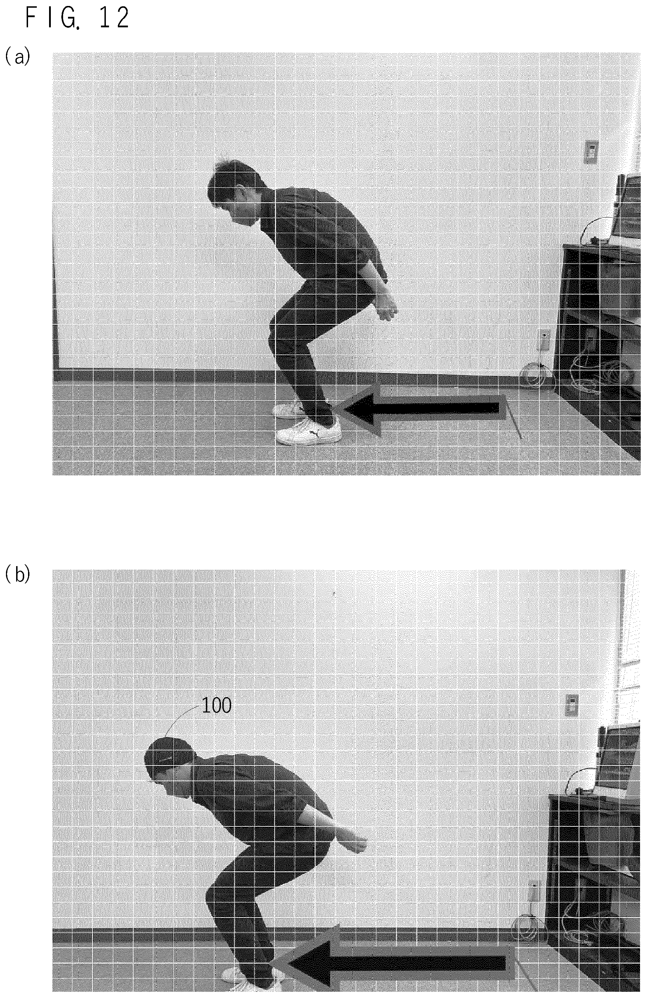

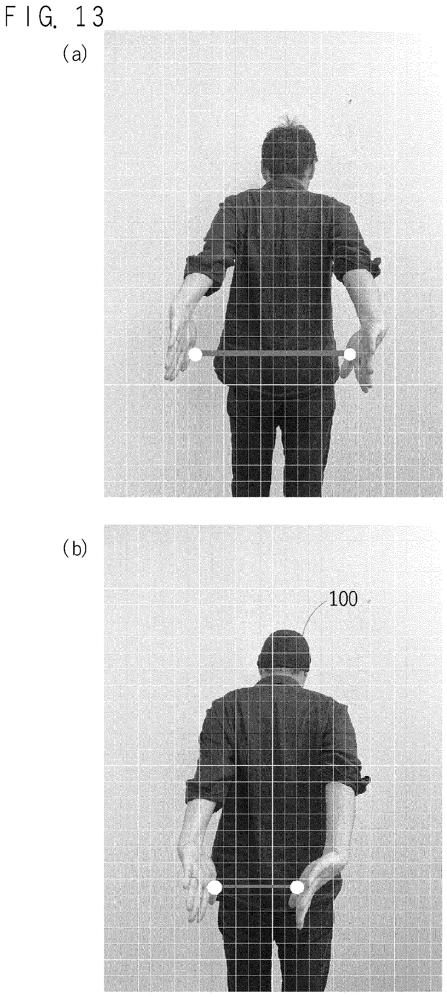

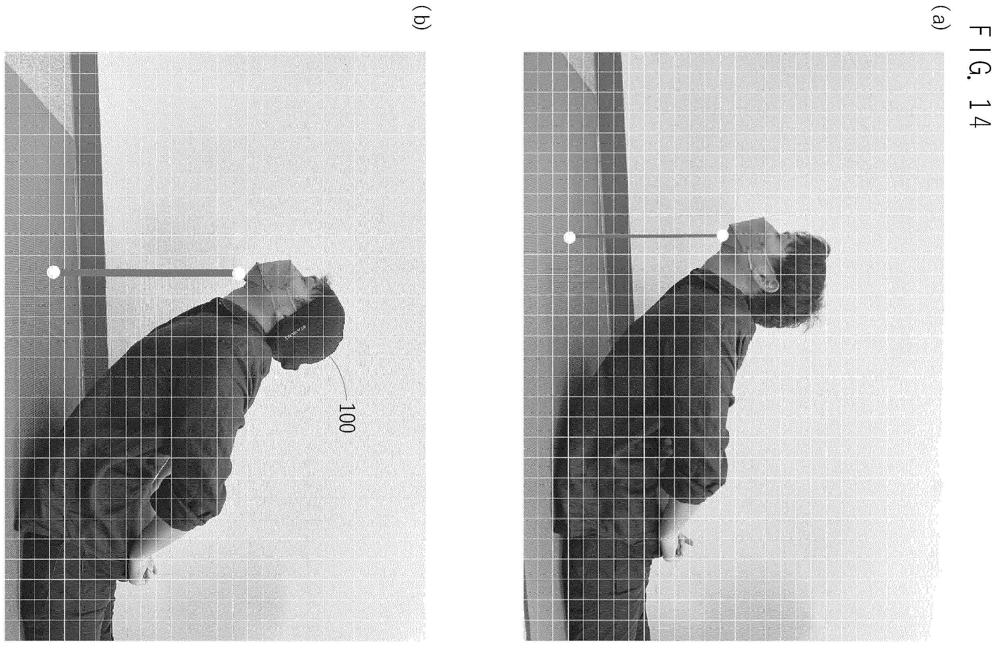

Next, the beneficial effects of embodying examples of instances in which a cap 100 of the present embodying modes was worn will be specifically described. Fig. 10 shows the results of a physical functioning/flexibility test when the cap 100 is worn. In turn, Fig. 11 (a) and (b) respectively show states of bending over forward when with and without the cap 100. Fig. 12 (a) and (b) respectively show states of a crouched-stance leap when with and without the cap 100. Fig. 13 (a) and (b) respectively show states of rearward arm swing when with and without the cap 100. Fig. 14 (a) and (b) respectively show states of a trunk lift when with and without the cap 100.

Fig. 10 is a table showing the results of before (comparison example "pre-wear") and after (embodying example "post-wear") having test subjects put on a cap 100 of the present embodying modes. The test subjects were eleven persons, No. 1 to No. 11.

As shown in Fig. 11 (a) and (b), "forward bend" indicates the angle (and the change between pre- and post-wear) when a forward bend is made. "Forward bend" is, in order to secure more objectivity than it being measured by hand, the angle in a posture analysis report in which images were captured/analyzed with a posture analysis app (an Android-version smartphone app) produced by SysNavia K.K. Forward-bend results are, for illustrating flexibility, easy to understand; with the embodying example of Fig. 11(b), compared with the comparison example of Fig. 11(a), the result that flexibility is improved can be apprehended. As shown in the table of Fig. 10, the average value (cm) of the comparison examples was 4.7, whereas the average value (cm) of the embodying examples was 0.4, wherein a remarkable as much as -4.3 (cm) result was shown, clearly indicating that flexibility improved.

With Fig. 12 (a) and (b), states of a crouched-stance leap in which a jump is made from a situation of being on one's feet at a standstill are shown. The results of the crouched-stance leap are, for illustrating improvement in athletic characteristics (exercise capacity), easy to understand; with the embodying example of Fig. 12(b), compared with the comparison example of Fig. 12(a), the result that athletic characteristics (exercise capacity) is improved can be apprehended. As shown in the table of Fig. 10, the average value (cm) of the comparison examples was 143.7, whereas the average value (cm) of the embodying examples was 153.1, wherein an as much as +8.7 (cm) remarkable result was shown, clearly indicating that athletic characteristics (exercise capacity) improved.

With Fig. 13 (a) and (b), states of rearward arm swings are shown. "Rearward arm swing" is, in order to secure more objectivity than it being measured by hand, the dimension in a posture analysis report in which images were captured/analyzed with the posture analysis app. Rearward arm-swing results are, for illustrating flexibility, easy to understand; with the embodying example of Fig. 13(b), compared with the comparison example of Fig. 13(a), the result that flexibility improved can be apprehended. As shown in the table of Fig. 10, the average value (cm) of the comparison examples was 33.3, whereas the average value (cm) of the embodying examples was 18.8, wherein a remarkable as much as -14.5 (cm) result was shown, clearly indicating that flexibility improved.

With Fig. 14 (a) and (b), states of trunk lift are shown. "Trunk lift" is, in order to secure more objectivity than it being measured by hand, the dimension in a posture analysis report in which images were captured/analyzed with the posture analysis app. Trunk lift results are, for illustrating flexibility, easy to understand; with the embodying example of Fig. 14(b), compared with the comparison example of Fig. 14(a), the result that flexibility improved can be apprehended. As shown in the table in Fig. 10, the average value (cm) of the comparison examples was 36.7, whereas the average value (cm) of the embodying examples was 42.5, wherein a remarkable as much as +5.8 (cm) result was shown, clearly indicating that flexibility improved.

In this way, even with just a glance at the averages for the eleven test subjects, improvement in flexibility as well as exercise capacity was clearly confirmed. And taking a look at not the average figures but the individual test subjects, some who showed remarkable improvement variously appear.

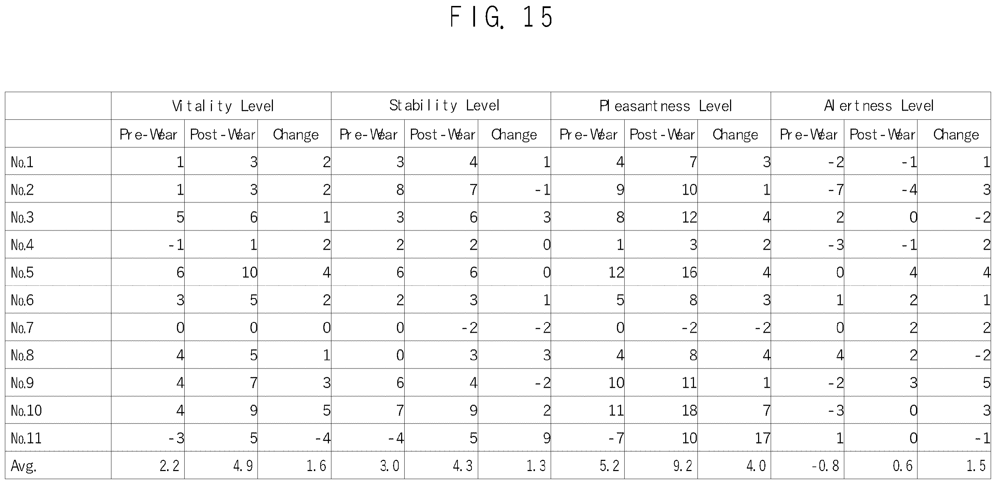

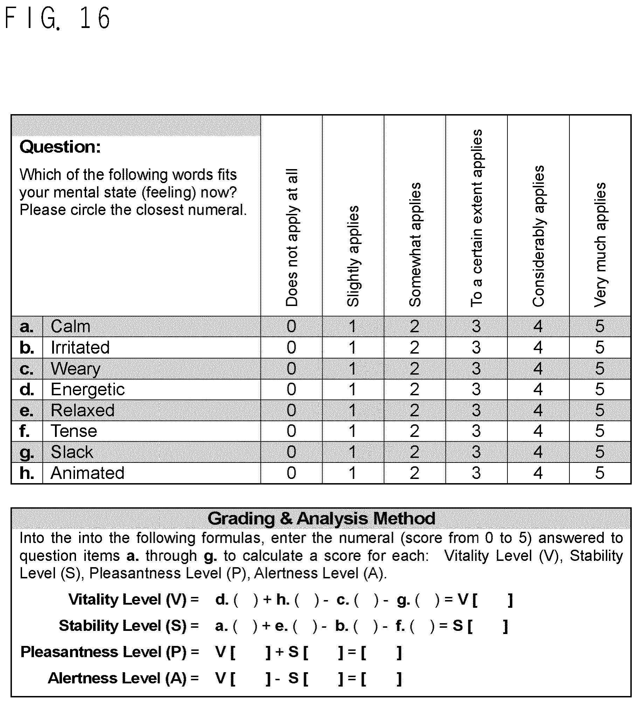

Fig. 15 is a table setting forth results of activity-level/stability-level/comfort-level/alertness-level tests that reveal psychological state (feeling) when the cap 100 is being worn. That is, "Activity Level," "Stability Level," "Comfort Level," and "Alertness Level" in Fig. 15 are items whose indices (two-dimensional gauges of feeling) cannot be captured in numeric figures as to improvement in capacity for movement. These "two-dimensional feeling gauges" are according to the paper, "Psychological Effects That Wearing Compression Garment for Sports Exert" in Hosei University Physical Education & Sports Center Proceedings, Tadashi Nakazawa (Housei University) et al., Vol. 30, pp. 29-34 (2012). A questionnaire (in item-scoring form) for the two-dimensional feeling gauges is as presented in Fig. 16.

Looking at the results for (two-dimensional feeling gauges of) "Activity Level," "Stability Level," "Comfort Level," and "Alertness Level" in Fig. 15-on average, respectively-activity level, a 1.6-point rise; stability level, a 1.3-point rise; comfort level, a 4.0-point rise; and alertness level, a 1.5-point rise: with all the items the indices improved. Accordingly, with the two-dimensional feeling gauges as well, the beneficial effects of a cap 100 of the present embodying example were confirmed.

Next, a modified example of a cap 100 of the present embodying modes will be described. With the cap 100 depicted in Fig. 1 to Fig. 3, the head-area patterned piece 20 is structured by means of the tape medium 21, but the head-area patterned piece 20 can be constituted from an ink-impressed figure, with the ink-impressed figure containing powder of a mineral(s).

With a preferred instance of the modified example, the ink-impressed figure(s) constituting the head-area patterned piece 20 is formed by silkscreen printing. In implementations with an ink-impressed figure(s), compared with those fabricated utilizing a tape medium, fabrication by printing (especially, fabrication by silk-screen printing) is better suited to manufacturing in small-lot as well as massive-lot production, wherein manufacturing costs can thus be kept under control. Furthermore, in tape-material implementations, repeated laundering or the like could lead to its peeling off; in order to prevent that, sewing the tape medium fast to the cap with thread is desirable, but doing so ends up raising manufacturing costs. By comparison with that, constituting the head-area patterned piece 20 with an ink-impressed figure in a cap 100 of the present embodying modes minimalizes peeling-off of the tape medium, enabling it to be made long-lasting, and at the same time makes it possible to keep manufacturing costs in check, since the work of sew-fastening with thread is not needed. It should be noted that in implementations that constitute the head-area patterned piece 20 with an ink-impressed figure(s), not providing the mesh section 25 in the mid-location along the cap body element 10 is preferable, as printing is facilitated. It will be appreciated that providing the mesh section 25 in a location(s) apart from where the head-area patterned piece 20 (ink-impressed figure(s)) is formed would not be a problem in particular.

As inks in silkscreen printing for prints on cap, plastisols (inks), and aqueous rubbers (inks) can be cited. With factories that carry out large-volume printing (large-lot projects of 300-plus printings) plastisols are often used, while with factories of medium scale (small-lot projects on the order of 50 printings) aqueous inks are often used. It is to be noted that with headgear (a cap 100) of the present embodying mode, silkscreen printing is utilized, but as long as the beneficial effects of headgear (a cap 100) of the present embodying modes can be obtained, other printing (e.g., inkjet direct printing, etc.) may be employed. Further, the printing ink (ink for prints on the cap) of the present embodying mode is compounded with mineral(s) (powder). With one example, printing ink of the present embodying mode is adjusted at a concentration in which a mineral(s) (a mineral that is referred to as a so-called healing crystal (e.g., radium ore, germanium, quartz, "terahertz ore," tourmaline, etc.), or a blend of minerals of several kinds) whose particle size is 325 mesh (about 44 microns)-in which per 500 square centimeters of ink, the appropriate amount among 0.1 g to 0.5 g of each kind (with single examples, one kind, two kinds, or three kinds)-is compounded. It is to be noted that 500 square centimeters of ink is a surface area equivalent to the print surface area on one cap. It is also to be noted that this concentration is a single example; changing/adjusting it to what is suitable and appropriate to accord with the conditions under which the ink is used is possible. Furthermore, additives apart from minerals (powders) may be included as may suit. A powder of a mineral(s) of at least one kind (or an ore powder blend of two or more kinds) selected from the group consisting of radium ore, germanium, quartz, terahertz ore, and tourmaline can be utilized as the mineral powder.

Due to differences arising between the fabric area of the cap body element 10, and the impressed area (head-area patterned piece 20) in tactile feel and other properties (difference in texture, differences in softness, elasticity, surface smoothness, and other properties of the material), furthermore, the differences in properties, activating given regions, can assist (reinforce, amplify) flexibility/capacity for movement. It should be noted that though the differences in these properties may be slight, it is not that the ink-impressed figure(s) (head-area patterned piece 20) supports a given location(s) to accomplish the function of improvement in capacity for movement; (inasmuch as the body is a cluster of delicate sensors) that it occasions stimulation is enough-even if the difference in properties due to the presence of the ink-impressed figure(s) is slight, beneficial effects of improving flexibility/exercise capacity can be demonstrated. And like it is a fact that acupuncture and moxibustion performed on the head area can have an effect on the body, a cap 100 of the present embodying modes, simply by being worn on the head, can improve the wearer's physical functioning and flexibility besides. There is a likelihood, furthermore, that by a mineral(s) being compounded into the ink-impressed figure(s) (head-area patterned piece 20), influences from the mineral(s) (e.g., negative ions, terahertz waves, static electricity, far infrared rays, hormesis, etc.) amount to stimuli on a given location(s), while thereby there is a likelihood that the beneficial effects from a cap 100 of the present embodying modes are reinforced.

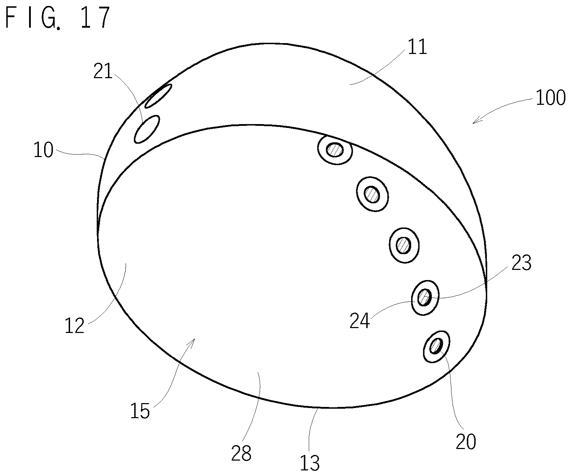

Next, referring to Fig. 17, a description of a further modified example of the cap 100 of the present embodying modes will be made. With the cap 100 depicted in Fig. 1 through Fig. 3, a rectangularly shaped head-area patterned piece 20 is formed, but as depicted in Fig. 17, the head-area patterned piece 20 may be structured from mineral buttons 23 provided on at least one of the reverse surface 12 or the obverse surface 11 of the cap body element 10. The mineral buttons 23 can be plurally arrayed along the head-area centerline (C). With the configuration of the present embodying mode, the mineral buttons 23 constituting the head-area patterned piece 20 are composed in such a way as to be in symmetrical form along the head-area centerline (C).

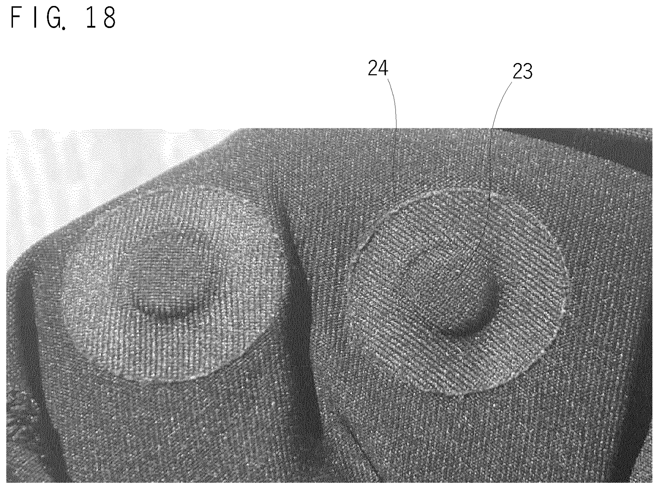

In the example represented in Fig. 17, the mineral buttons 23 are formed on the reverse surface 12 of the cap body element 10. The mineral buttons 23 with this example are circular articles in the interiors of which a mineral(s) is housed. It should be noted that the mineral buttons 23 may be of shapes other than circular (e.g., quadrilateral, hexagonal, etc.), but it being preferable that they are circular is often the case. With the diagrammed instance, the mineral buttons 23 are arrayed equally spaced along the head-area centerline (C). The mineral buttons 23 have a part 24 in the interior of which a mineral(s) (e.g. a lump of a mineral(s), a powdered mass of a mineral(s), or an article in which a powdered mass of a mineral(s) has been rendered into a button) is fixed. Fig. 18 is an enlarged picture showing the structure of mineral buttons 23, wherein the part 24 in the interior of which a mineral(s) is fixed extends to the periphery in a way such as to cover the mineral button 23. The part 24 in the interior of which a mineral(s) is fixed is, for example, anchored to the reverse surface 12 of the cap body element 10 by being pressure-adhered (welded or bonded) to make it cover the mineral button 23. The mineral(s) in the mineral buttons 23, furthermore, is of a kind as described above: a mineral that is referred to as a so-called healing crystal (e.g., radium ore, germanium, quartz, terahertz ore, tourmaline, etc.) or a blend of minerals of several kinds.

While in the foregoing, the present invention has been described according to preferred embodying modes, the description thus rendered is not a limiting matter; inarguably various modifications are possible. The above-described embodying modes as well as modified-example configurations as well as techniques are applicable to one another. The cap body element 10 of the present embodying modes (cap 100), with the main focus being on having it fit on securely, has been described as being rendered a structure/form like that of a swimming cap, but it may be an article of the sort that has a brim. In particular, when for playing golf or the like a cap (headgear) of the sort that has a brim is preferred, it can be modified into an article of design of that sort. And otherwise, there is nothing to prohibit putting other headgear on atop a swimming-cap sort of cap 100 that has been put on. It may be employed not only as headgear that is used on land, but also as a swimming cap. What is more, as long as the beneficial effects, including flexibility, in the embodying modes of the present invention can be gained reliably, the head-area patterned piece 20 may be formed on the obverse surface 11 rather than the reverse surface 12 of the cap body element 10; alternatively, head-area patterned pieces 20 may be formed on both the reverse surface 12 and the obverse surface 11 of the cap body element 10. Furthermore, although with the head-area patterned piece 20 depicted in Fig. 1 as well as Fig. 2, an implementation of rectangular form (or approximately rectangular form) is indicated, the outer edges (left and right sides) 22 that define the shape of the head-area patterned piece 20 may be non-rectilinear (including curved lines or wavy forms). What is more, other suitable modifications can be made as appropriate.

### INDUSTRIAL APPLICABILITY

According to the present invention, headgear (a cap) that can improve the wearer's physical functioning (athletic functioning) as well as flexibility can be made available.

### DESCRIPTION OF REFERENCE NUMERALS

- 10:: cap body element
- 11:: cap body element obverse surface
- 12:: cap body element reverse surface
- 13:: rim part (mouth rim part)
- 15:: opening
- 20:: head-area patterned piece
- 21:: tape medium
- 22:: outer edges (left side/right side)
- 23:: mineral button
- 25:: mesh section
- 29:: elastic section
- 30:: mineral patterning
- 51:: first patch
- 52:: second patch
- 100:: cap (headgear)

## Claims

1. A cap for being worn on a head, the cap comprising:
a cap body element for covering an area of the head; and
a head-area patterned piece extending in the cap body element along a centerline of the head area; wherein
the head-area patterned piece is constituted from a tape medium adhered to at least one of reverse and obverse surfaces of the cap body element, and
the tape medium in the head-area patterned piece contains a mineral(s).

2. The cap set forth in claim 1, wherein:
the mineral(s) is at least one type of mineral selected from the group consisting of radium ore, germanium, quartz, terahertz ore, and tourmaline;
the mineral(s) is invested into the tape medium by being printed onto it; and
the mineral(s) invested by the printing have a pattern form selected from the group consisting of geometric shapes, design marks, and written characters.

3. The cap set forth in claim 1 or 2, wherein:
on one region of the tape medium a first patch constituted from a first metal is formed;
on another region of the tape medium a second patch constituted from a second metal is formed; and
the first patch is a gold patch, and therein the second patch is a silver patch.

4. The cap set forth in claim 1 or 2, wherein:
on one region of the tape medium a first patch constituted from a first metal is formed;
on another region of the tape medium a second patch constituted from a second metal is formed; and
the first patch is either a patch composed of an alloy of zinc and copper, or a copper patch, and therein the second patch is an aluminum patch.

5. The cap set forth in claim 1 or 2, wherein the head-area patterned piece is formed on the reverse face of the cap body element.

6. The cap set forth in claim 1 or 2, wherein:
the head-area patterned piece has a rectangular form;
a rim part that defines an opening on the head-area patterned piece constitutes an elastic section;
on the cap body element, a mesh section that extends along the head-area centerline is formed; and
the head-area patterned piece is formed on an inner-surface side of the mesh section.

7. The cap set forth in claim 1 or 2, wherein:
the cap body element is constituted from a stretchable material;
the head-area patterned piece is of 25 mm ±10 mm width; and
the head-area patterned piece is formed in such a way as to be symmetrical with respect to the head-area centerline.

8. A cap for being worn on a head, the cap comprising:
a cap body element for covering an area of the head; and
a head-area patterned piece extending in the cap body element along a centerline of the head area; wherein
the head-area patterned piece is constituted from an ink-impressed figure(s) formed on at least one of reverse and obverse surfaces of the cap body element; and
the ink-impressed figure(s) contain powder of a mineral(s).

9. The cap set forth in claim 8, wherein:
the mineral(s) is at least one type of mineral selected from the group consisting of radium ore, germanium, quartz, terahertz ore, and tourmaline; and
the ink-impressed figure(s) have a pattern form selected from the group consisting of geometric shapes, design marks, and written characters.

10. The cap set forth in claim 8 or 9, wherein:
on one region of the head-area patterned piece a first patch constituted from a first metal is formed;
on another region of the head-area patterned piece a second patch constituted from a second metal is formed; and
the first patch is a gold patch, and therein the second patch is a silver patch.

11. The cap set forth in claim 8 or 9, wherein:
on one region of the head-area patterned piece a first patch constituted from a first metal is formed;
on another region of the head-area patterned piece a second patch constituted from a second metal is formed; and
the first patch is either a patch composed of an alloy of zinc and copper, or a copper patch, and therein the second patch is an aluminum patch.

12. The cap set forth in claim 8 or 9, wherein:
the head-area patterned piece is formed on the reverse face of the cap body element;
the cap body element is constituted from a stretchable material;
the width of the head-area patterned piece is 25 mm ±10 mm; and
the head-area patterned piece is formed in such a way as to be symmetrical with respect to the head-area centerline.

13. A cap for being worn on a head, the cap comprising:
a cap body element for covering an area of the head; and
a section of head-area patternings in the cap body element, arrayed along a centerline of the head area; wherein
the head-area patternings section has mineral buttons provided on at least one of reverse and obverse surfaces of the cap body element; and
the mineral buttons are plurally arrayed along the head-area centerline.

14. The cap set forth in claim 13, wherein:
the mineral buttons are formed on the reverse face of the cap body element;
the mineral buttons are circular articles interiorly housing a mineral(s); and
the mineral buttons are arrayed evenly spaced apart along the head-area centerline.

15. A cap manufacturing method comprising:
a step of preparing a cap a body element for covering a head area; and
a step of forming, in the head-area-covering cap body element along a centerline of the head area, head-area patterned pieces in which a mineral(s) is contained.

16. The cap manufacturing method set forth in claim 15, wherein along the centerline, adhering of a tape medium in which the mineral(s) is contained is carried out.

17. The cap manufacturing method set forth in claim 15, wherein in the step of forming the head-area patterned pieces, forming of an ink-impressed figure(s) by applying an ink in which the mineral(s) is contained is carried out.

18. The cap manufacturing method set forth in claim 15, wherein in the step of forming the head-area patterned pieces, plural-item forming, along the centerline, of mineral buttons interiorly housing the mineral(s) is carried out.

19. The cap manufacturing method set forth in any one of claims 15 through 18, further wherein on one region of the head-area patterned pieces, forming of a first patch constituted from a first metal, and then on another region of the head-area patterned pieces, forming of a second patch constituted from a second metal are carried out.
